# EUROPEAN PATENT APPLICATION

(11) **EP 2 305 095 A1**
(43) Date of publication of application: **06.04.2011**
(21) Application number: 10182265.8
(22) Date of filing: 29.09.2010
(51) Int. Cl.: A61B 1/04, A61B 1/05, A61B 1/00, G02B 21/02, G02B 27/00, G02B 23/24

(54) **Imaging apparatus**

(30) Priority: 30.09.2009 JP 2009226697
(71) Applicant: Fujifilm Corporation, Minato-ku Tokyo (JP)
(72) Inventor: Ono, Shuji, Kanagawa (JP)
(74) Representative: Klunker . Schmitt-Nilson . Hirsch

(57) **Abstract**

An imaging apparatus includes an illumination light output system (10, 11, 12, 14) that outputs light (L) including wavelength components that are different from each other to living body tissue (31), an image formation optical system (20) that forms an image of the living body tissue (31) illuminated with the light output from the illumination light output system (10, 11, 12, 14), and an imaging means (23) that images the living body tissue (31) . The image formation optical system (20) has axial chromatic aberration in which focal length for C line that has a wavelength of 643.8 nm is greater than or equal to 102 when focal length for F line that has a wavelength of 486.1 nm is regarded as 100.

## Description

### Field of the Invention

The present invention relates to an imaging apparatus, and particularly to an imaging apparatus for imaging living body tissue, such as an endoscopic apparatus.

### Description of the Related Art

Conventionally, endoscopic apparatuses were widely used to observe the inside of a living body or to treat the inside of the living body while observing it. Currently, so-called electronic endoscopes are mainly used as the endoscopic apparatuses. An electronic endoscope includes an illumination light output system, an image formation optical system and an imaging means. The illumination light output system outputs illumination light to a region in the inside of the living body through an optical fiber or the like. The image formation optical system forms an image by the illumination light reflected from the region. The imaging means captures the image formed by the image formation optical system. Examples of such endoscopic apparatuses are described in U. S. Patent Application Publication No. 20030139650 (Patent Document 1) and U.S. Patent No. 7,678,045 (Patent Document 2).

As the illumination light output system, a system that outputs white light, a system that sequentially outputs light in red (R) region, light in green (G) region, and light in blue (B) region, and the like are adopted. In observation of living body tissue, the appropriate wavelength of illumination light differs depending on the position of a region to be observed (a target of observation) in the depth direction of the living body tissue. The longer the wavelength of illumination light is, the deeper the illumination light reaches in the living body tissue. Specifically, illumination light of short wavelength in blue region reaches only a part of the living body tissue that is relatively close to the surface of the living body tissue. However, illumination light of long wavelength, for example, in red region reaches a deeper part of the living body tissue. For example, when blood vessels in living body tissue are considered, capillary vessels are present relatively close to the surface of the living body tissue, and thicker blood vessels are present at deeper positions of the living body tissue. Therefore, when capillary vessels are observed, illumination light of blue region is desirable. However, when a deep part in which thick blood vessels are present is observed, illumination light of red region is desirable.

Under such circumstances, conventionally, various endoscopic apparatuses that can obtain appropriate information about living body tissue for a wide range in the depth direction thereof by changing the properties of illumination light based on the position of the living body tissue to be observed were proposed. For example, Patent Document 1 proposes an endoscopic apparatus in which a blue filter, a green filter and a red filter is selectably inserted into the optical path of illumination light. In the endoscopic apparatus, when a shallow layer portion, which is close to the surface of the living body tissue, a middle layer portion and a deep layer portion of the living body tissue are imaged, illumination light that has passed through a blue filter, a green filter and a red filter, respectively, is output to the portions to be observed.

Further, Patent Document 1 proposes a structure in which a flat portion is provided at the pupil of an image formation optical system in such a manner that the center of the flat portion and the periphery of the flat portion have different spectral transmission characteristics from each other. Patent Document 1 can obtain a sufficient light amount for a wavelength band (green to blue) for which the area of the pupil has been increased and high frequency transmission characteristics. However, the focal depth becomes small if the area of the pupil is merely increased. Therefore, the focal depth is increased by using a phase mask and by performing signal processing.

Further, Patent Document 2 proposes an endoscopic observation optical system in which a magnification ratio for blood vessel emphasized observation is set higher than a magnification ratio for visible light ordinary observation. In Patent Document 2, the axial chromatic aberration in the observation optical system is insufficiently corrected for light having a wavelength of 415 nm in such a manner that the axial chromatic aberration remains, and a focused object position in blood vessel emphasized observation is set on the near-point side of the focused obj ect position in visible light ordinary observation. Accordingly, the magnification ratio for the blood vessel emphasized observation is set higher than the magnification ratio for the visible light ordinary observation.

However, in an endoscopic apparatus in which a plurality of of filters are inserted in the optical path of illumination light in a selectable manner, the structure of the endoscopic apparatus becomes complex, because a mechanism for moving the filters is provided. Further, the cost becomes higher. Such problems may occur not only in the endoscopic apparatus but also in other imaging apparatuses that image living body tissue illuminated with light including different wavelength components from each other.

Meanwhile, in the endoscopic apparatus in which the flat portion is provided at the pupil of the image formation optical system in such a manner that the center of the flat portion and the periphery of the flat portion have different spectral transmission characteristics from each other, and in which the focal depth is increased by using the phase mask and by performing signal processing, it is impossible to efficiently obtain information. Specifically, in the structure of the endoscopic apparatus, the focal depth is increased at a true focused position toward the front side and the back side thereof. However, the living body tissue, which is the target of observation, is present in the range from the surface of the living body tissue to a deep part of the living body tissue. Therefore, even if the focal depth is increased at the surface of the living body tissue toward the front side (lens side), the amount of information obtained by imaging does not increase.

### SUMMARY OF THE INVENTION

In view of the foregoing circumstances, it is an object of the present invention to provide an imaging apparatus having simple structure that can obtain appropriate information about living body tissue for a wide range in the depth direction thereof.

An imaging apparatus according to the present invention is an imaging apparatus comprising:
an illumination light output system that outputs light including wavelength components that are different from each other to living body tissue;
an image formation optical system that forms an image of the living body tissue illuminated with the light output from the illumination light output system; and
an imaging means that images the living body tissue, wherein the image formation optical system has axial chromatic aberration in which focal length for C line that has a wavelength of 643.8 nm is greater than or equal to 102 when focal length for F line that has a wavelength of 486.1 nm is regarded as 100.

It is desirable that the image formation optical system is structured in such a manner that focal depth for illumination light that has a relatively long wavelength is greater than focal depth for illumination light that has a relatively short wavelength.

It is desirable that the imaging apparatus of the present invention further includes an image restoration means that performs image restoration processing on an image signal output from the imaging means so as to reduce a blur in the image caused by the image formation optical system.

The illumination light output system may be a system, such as a white light output system, for example. The system, such as the white light output system, outputs wavelength components that are different from each other to the living body at the same time. Alternatively, the illumination light output system may output wavelength components that are different from each other to the living body tissue separately at respective time points (sequentially or the like), in other words, in such a manner that different wavelength components are not output at the same time.

The axial chromatic aberration of the image formation optical system in the imaging apparatus of the present invention is extremely large, compared with the axial chromatic aberration of conventional image formation systems that are used in apparatuses for imaging living body tissue. Specifically, the axial chromatic aberration of the image formation optical system of the present invention is naturally larger than the axial chromatic aberration of a conventional image formation optical system including an achromatic lens, and which is applied to an endoscopic apparatus or the like in many cases. Further, the axial chromatic aberration of the image formation optical system of the present invention is also larger than the axial chromatic aberration of a conventional image formation optical system that does not have the achromatic structure. The image formation optical system that has such large axial chromatic aberration may be obtained, for example, by using a plurality of lenses that are used for a conventional achromatic lens optical system, and by arranging the plurality of lenses in a different manner from the arrangement of the achromatic lens optical system.

In the imaging apparatus of the present invention, in which the image formation optical system as described above is used, a focused position by illumination light that has a relatively short wavelength and a focused position by illumination light that has a relatively long wavelength are apart from each other by a larger distance, compared with an imaging apparatus, such as a conventional endoscopic apparatus. Therefore, in the imaging apparatus of the present invention, a part of the living body tissue close to the surface of the living body tissue is imaged by using illumination light of a relatively short wavelength in a sharply focused manner. Further, a deep part of the living body tissue is imaged by using illumination light of a relatively long wavelength in a sharply focused manner. Therefore, it is possible to obtain appropriate information for a wide range in the depth direction of the living body tissue.

The advantageous effects as described are achieved simply by adopting the image formation optical system, in which the axial chromatic aberration is increased compared with a conventional system. Such an image formation optical system is obtained by using a simple method as will be described later. Specifically, the type of glass material and a refractive index are combined with each other in a reverse manner to a combination thereof in an ordinary achromatic lens. Therefore, the advantageous effect of the imaging apparatus of the present invention is achieved without structuring the imaging apparatus in a complicated manner. The imaging apparatus of the present invention has simple structure, and can be produced at low cost.

In the imaging apparatus of the present invention, especially when the focal depth of the image formation optical system for illumination light that has a relatively long wavelength is greater than the focal depth of the image formation optical system for illumination light that has a relatively short wavelength, it is possible to image a deep part of the living body tissue in a wider range in the depth direction thereof to obtain a sharp image.

In the imaging apparatus of the present invention, the image formation optical system as described above is adopted. Therefore, a blur tends to be generated or increased in an image obtained by imaging, depending on the deterioration characteristic of the optical system. Therefore, if an image restoration means that performs image restoration processing on an image signal output from the imaging means is provided to reduce a blur in the image caused by the image formation optical system, it is possible to obtain a sharp image by removing the blur. As the image restoration processing, a method disclosed, for example, in Japanese Unexamined Patent Publication No. 2009-089082 is known. In the present invention, such a conventional image restoration processing method may be used.

The image restoration processing may be performed on all image signals output from the imaging means. Alternatively, when the focal depth of the image formation optical system for illumination light of a relatively long wavelength is greater than the focal depth of the image formation optical system for the illumination light of a relatively short wavelength, and when the illumination light output system outputs different wavelength components to the living body tissue separately at respective time points, the image restoration processing may be performed in such a manner that only image signals representing an image by illumination light of a relatively long wavelength, in which a blur tends to be generated or increased, are processed.

### BRIEF DESCRIPTION OF THE DRAWINGS

Figure 1 is a schematic diagram illustrating a side view of an endoscopic apparatus according to an embodiment of the present invention; and
Figure 2 is a graph illustrating the properties of an image formation optical system of the endoscopic apparatus according to the embodiment of the present invention together with the properties of other optical systems.

### DESCRIPTION OF THE PREFERRED EMBODIMENTS

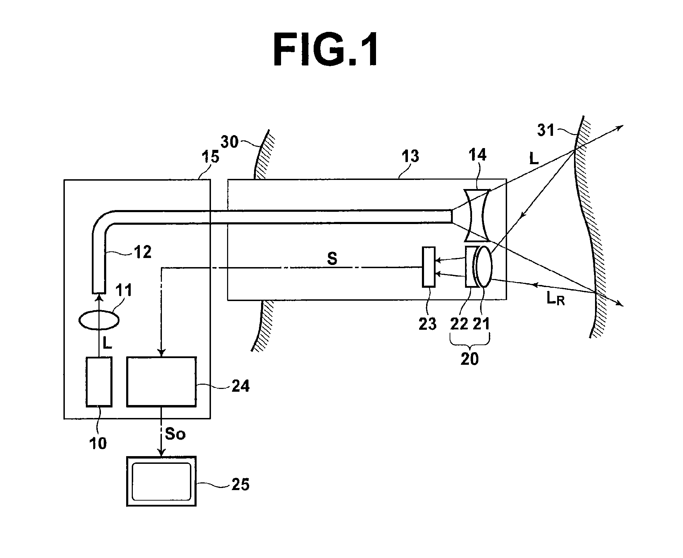

Hereinafter, embodiments of the present invention will be described with reference to drawings. Figure 1 is a schematic diagram illustrating a side view of an imaging apparatus according to an embodiment of the present invention. One of examples of the imaging apparatus is an endoscopic apparatus. The imaging apparatus includes an illumination light source 10 that outputs illumination light L, which is white light. Further, the imaging apparatus includes a condensing lens 11 that condenses the illumination light L, and a light guide 12. The light guide 12 includes an optical fiber that is arranged in such a manner that the condensed illumination light L enters an end of the optical fiber. Further, a portion of the light guide 12 that is close to the other end of the light guide 12 is housed in a flexible scope unit 13, which is introduced into the inside (body cavity or the like) of a living body 30. Further, in the scope unit 13, an illumination lens 14 is arranged at a position facing the other end of the light guide 12.

In the present embodiment, the illumination light output system includes the illumination light source 10, the condensing lens 11, the light guide 12, and the illumination lens 14. The illumination light source 10, the condensing lens 11, and a part of the light guide 12 that is close to the end thereof are arranged in a processor unit 15 together with an image processing circuit 24, which will be described later.

Further, an image formation optical system 20 is arranged in the scope unit 13. The image formation optical system 20 is arranged in the vicinity of an insertion side end of the scope unit 13 (right end of the scope unit 13 in Figure 1). The image formation optical system 20 includes, for example, two lenses 21 and 22. Further, a solid-state imaging device 23, such as a CCD imaging device (charge coupled device), is arranged on the rear side of the image formation optical system 20. The solid-state imaging device 23 is connected to the image processing circuit 24, and the image processing circuit 24 is connected to an image display means 25 including a CRT (cathode-ray tube) display device, or the like.

Next, the basic operation of the endoscopic apparatus structured as described above will be described. When a region (living body tissue) 31 in a living body 30 is observed, the scope unit 13 is introduced into the living body 30, for example, through the body cavity of a patient. Further, illumination light L is output from the light guide 12 to the region 31. The image formation optical system 20 forms, on an imaging plane of the solid-state imaging device 23, an image of the region 31 by illumination light L_{R} that has been reflected from the region 31. The solid-state imaging device 23 captures (images) the image formed by the image formation optical system 20, and inputs image signal S representing the image into the image processing circuit 24.

The image processing circuit 24 performs processing, such as image restoration processing which will be described later, on the image signal S to generate image signal So. The image processing circuit 24 sends the image signal So to the image display means 25. The image display means 25 displays an image of the region 31 based on the image signal So. Accordingly, a surgeon or doctor, or an assistant to the surgeon or doctor, or the like can observe an ordinary image of the region 31 of the living body at the image display means 25.

Next, the image formation optical system 20 will be described in detail. Here, the design of a lens optical system that has a focal length of 100 mm for F line (wavelength is 486.133 nm), more particularly, the design of a complex lens optical system including two thin lenses made of optical glass BK7 and SF2 respectively will be considered. The aforementioned focal length may be realized by combining the two kinds of optical glass in various manners. However, the following Table 1 shows typical three types and a positive lens that is made of BK7 alone.

**[Table 1]**

| TYPE | BK7 FOCAL LENGTH(mm) | SF2 FOCAL LENGTH (mm) |
|---|---|---|
| BK7 ALONE | 101.1 | - |
| BK7-SF2 ACHROMATIC TYPE | 50.045 | -100 |
| SF2-BK7 INCREASED CHROMATIC ABERRATION TYPE A | -100 | 50.727 |
| SF2-BK7 INCREASED CHROMATIC ABERRATION TYPE B | -50 | 33.757 |

In Table 1, the "ACHROMATIC TYPE" has an achromatic function by arranging a positive lens made of BK7 and a negative lens made of SF2 in this order from the light entering side. The focal length of each lens is shown in Table 1. In Table 1, the focal length of a positive lens is represented by a positive value, and the focal length of a negative lens is represented by a negative value.

In Table 1, the "INCREASED CHROMATIC ABERRATION TYPE A" and the "INCREASED CHROMATIC ABERRATION TYPE B" refer to types in which the axial color aberrations are intentionally increased, compared with the case of using the positive lens made of BK7 alone. In each of the "INCREASED CHROMATIC ABERRATION TYPE A" and the "INCREASED CHROMATIC ABERRATION TYPE B", a positive lens made of SF2 and a negative lens made of BK7 are arranged in this order from the light entering side.

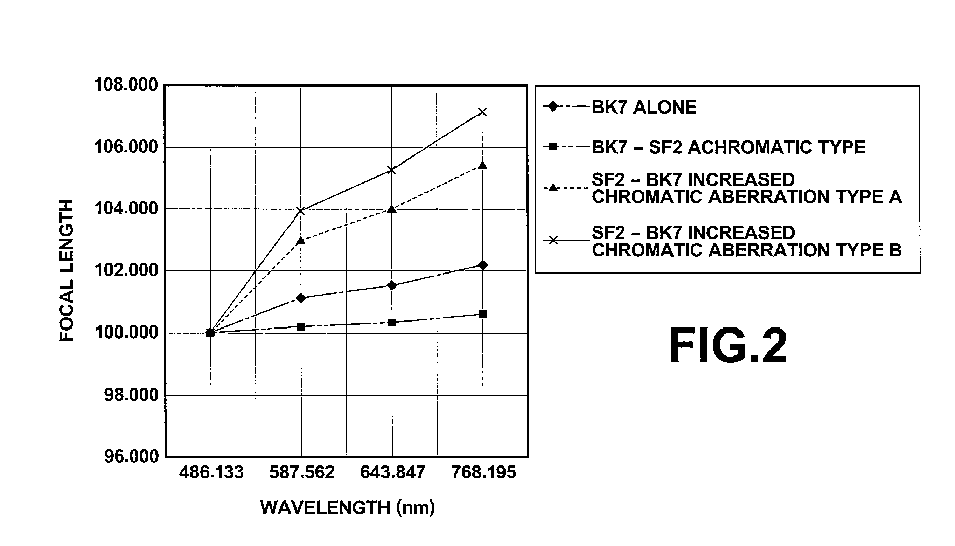

The following table 2 and Figure 2 illustrate the focal length of each of the four types of optical systems for F line (wavelength is 486.133 nm), d line (wavelength is 587.562 nm), C line (wavelength is 643.847 nm) and A' line (wavelength is 768.195 nm).

**[Table 2]**

| | WAVELENGTH (nm) | BK7 ALONE | BK7-SF2 ACHROMATIC TYPE | SF2-BK7 INCREASED CHROMATIC ABERRATION TYPE A | SF2-BK7 INCREASED CHROMATIC ABERRATION TYPE B |
|---|---|---|---|---|---|
| F LINE | 486.133 | 100.009 | 100.005 | 100.000 | 100.002 |
| d LINE | 587.562 | 101.101 | 100.180 | 102.951 | 103.912 |
| C LINE | 643.847 | 101.508 | 100.298 | 103.965 | 105.251 |
| A' LINE | 768.195 | 102.150 | 100.570 | 105.397 | 107.115 |

AS described above, the imaging apparatus of the present invention uses the image formation optical system that has axial chromatic aberration in which the focal length for C line (wavelength of 643.8 nm) is greater than or equal to 102 when the focal length for F line (wavelength of 486.1 nm) is regarded as 100. The optical systems of the "INCREASED CHROMATIC ABERRATION TYPE A" and the "INCREASED CHROMATIC ABERRATION TYPE B" satisfy the aforementioned condition of the axial chromatic aberration. In the structure illustrated in Figure 1, the optical system of the "INCREASED CHROMATIC ABERRATION TYPE A" is applied to the image formation optical system 20 as an example. Specifically, the lens 21 included in the image formation optical system 20 is a positive lens that is made of SF2 and has a focal length of 50.727 mm. The lens 22 included in the image formation optical system 20 is a negative lens that is made of BK7 and has a focal length of - 100 mm. Instead of the optical system of the "INCREASED CHROMATIC ABERRATION TYPE A", an optical system of the "INCREASED CHROMATIC ABERRATION TYPE B" may be applied to the image formation optical system 20.

As Figure 2 illustrates, in optical systems that have increased chromatic aberrations as described above, the focal length tends to be longer for light of longer wavelength. This tendency is observed naturally, compared with an achromatic lens system. Further, the tendency is also clear, even compared with an ordinary lens.

When the inside of living body tissue is observed from the outside of the living body tissue, visible light reaches only a position at the depth of approximately 0.3 mm or less from the surface of the living body tissue. Therefore, it is desirable that the focused position is set at approximately 0 mm from the surface of the living body tissue for the visible light. Meanwhile, near-infrared light reaches a position at the depth of 6 mm or more from the surface of the living body tissue. Therefore, it is desirable that the focused position is set at approximately 6 mm (in the vicinity of the point at the depth of 6 mm) from the surface of the living body tissue for the near-infrared light. For example, when near-infrared light having wavelength of 768 nm is used as illumination light, the "INCREASED CHROMATIC ABERRATION TYPE A" optical system is focused at the depth of 5 mm from the surface of the living body tissue, and the "INCREASED CHROMATIC ABERRATION TYPE B" optical system is focused at the depth of 7 mm from the surface of the living body tissue. As described above, the endoscopic apparatus of the present invention has a focal position characteristic that is appropriate for observing the inside of the living body tissue. The endoscopic apparatus of the present invention can obtain, for example, a sharp image of visible light and a sharp image of near-infrared light at the same time. In contrast, when a simple lens or an achromatic lens is used in an image formation optical system of an endoscopic apparatus, it is necessary to change the focused position based on the depth of a target position. Consequently, the structure and mechanism of the apparatus becomes complex.

In the endoscopic apparatus of the present embodiment, when the image formation optical system 20 as described above is adopted, a blur tends to be generated or increased in an image obtained by imaging, depending on the deterioration characteristic of the image formation optical system 20. Therefore, the image processing circuit 24 illustrated in Figure 1 performs image restoration processing on image signal S output from the solid-state imaging device 23 so as to reduce the blur. Accordingly, the blur caused by the deterioration characteristic of the image formation optical system 20 is reduced, and a sharp image is displayed. As the image restoration processing, a method disclosed, for example, in Japanese Unexamined Patent Publication No. 2009-089082 is known. The image processing circuit 24 in the present embodiment may be structured to perform such conventional image restoration processing.

In the above embodiment, the amount of shifting the focal length or position, in other words, a difference in the focal length for each wavelength or wavelength component is controlled by changing the refractive index of each lens. In the present embodiment, the properties of the increased chromatic aberration type, which are contrary to those of the achromatic type, are realized by combining the type of glass material and a refractive index for each of two lenses in a reverse manner to a combination of the type of glass material and a refractive index in an ordinary achromatic lens. However, the method for realizing the properties of the increased chromatic aberration type is not limited to this method. For example, even when the refractive index of each lens is further increased, similar properties may be realized by combining a larger number of lenses or various kinds of lens materials with each other so that the complex lens has focal length similar to the present embodiment.

The embodiment of the present invention structured as the endoscopic apparatus has been described. However, the present invention is not limited to the endoscopic apparatus. For example, the present invention may be applied to an imaging apparatus for measuring an image of a living body, such as a face and skin. Further, the present invention may be applied to an imaging apparatus for measuring blood flow velocity, or the like. Further, the present invention may be applied to an apparatus for imaging living body tissue that includes a general digital camera structure.

## Claims

1. An imaging apparatus comprising:
an illumination light output system (10, 11, 12, 14) that outputs light (L) including wavelength components that are different from each other to living body tissue (31);
an image formation optical system (20) that forms an image of the living body tissue (31) illuminated with the light (L) output from the illumination light output system (10, 11, 12, 14); and
an imaging means (23) that images the living body tissue (31), wherein the image formation optical system (20) has axial chromatic aberration in which focal length for C line that has a wavelength of 643.8 nm is greater than or equal to 102 when focal length for F line that has a wavelength of 486.1 nm is regarded as 100.

2. An imaging apparatus, as defined in Claim 1, wherein the image formation optical system (20) is structured in such a manner that focal depth for illumination light that has a relatively long wavelength is greater than focal depth for illumination light that has a relatively short wavelength.

3. An imaging apparatus, as defined in Claim 1 or 2, further comprising:
an image restoration means (24) that performs image restoration processing on an image signal (S) output from the imaging means (23) so as to reduce a blur in the image caused by the image formation optical system (20).

4. An imaging apparatus, as defined in any one of Claims 1 to 3, wherein the illumination light output system (10, 11, 12, 14) outputs the wavelength components that are different from each other to the living body tissue (31) at the same time.

5. An imaging apparatus, as defined in any one of Claims 1 to 3, wherein the illumination light output system (10, 11, 12, 14) outputs the wavelength components that are different from each other to the living body tissue (31) separately at respective time points.
